(19) Europäisches Patentamt
European Patent Office
Office européen des brevets

(11) Publication number: 0 166 377
A1

# (12) EUROPEAN PATENT APPLICATION

(21) Application number: **85107633.1**

(22) Date of filing: **20.06.85**

(51) Int. Cl.⁴: **C 07 D 499/10**, C 07 D 499/78, C 07 D 501/20, C 07 D 501/04, C 07 D 498/04, C 07 D 471/04 // (C07D498/04, 265:00, 205:00), (C07D471/04, 221:00, 205:00)

(30) Priority: **29.06.84 GB 8416648**

(43) Date of publication of application: **02.01.86**
**Bulletin 86/1**

(84) Designated Contracting States: **AT BE CH DE FR GB IT LI LU NL SE**

(71) Applicant: **BEECHAM GROUP PLC, Beecham House Great West Road, Brentford Middlesex TW8 9BD (GB)**

(72) Inventor: **Moores, Clive James, "Faraway Cottage" Trapp Lane, Walliswood Dorking Surrey RH5 5QX (GB)**

(74) Representative: **Lockwood, Barbara Ann et al, Beecham Pharmaceuticals Biosciences Research Centre Great Burgh Yew Tree Bottom Road, Epsom Surrey KT18 5XQ (GB)**

(54) **Process for the preparation of beta-lactam compounds and intermediates therefor.**

(57) A process for preparing a compound of partial structure (I):

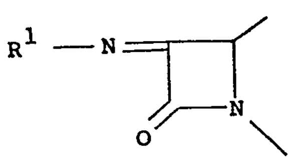

(I)

wherein $R^1$ is a carboxylic acyl group by reacting a compound of partial structure (II):

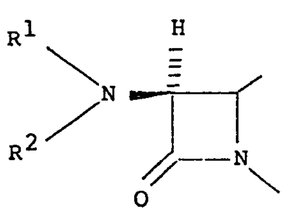

(II)

wherein $R^2$ is halogen with a non-nucleophilic organic base.

The compound of partial structure (I) can be converted in situ to a compound of partial structure:

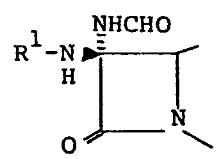

by addition of a nucleophilic derivative of formamide.

EP 0 166 377 A1

TITLE MODIFIED
see front page

CHEMICAL PROCESS

This invention relates to a chemical process for the preparation of β-lactam compounds and intermediates therefor, which process is particularly useful when a formamido substituent is to be introduced into a β-lactam at the carbon atom adjacent to the carbonyl group in the β-lactam ring. Certain of the substituted β-lactam compounds produced by the process are antibacterial agents; others are useful as intermediates for producing such agents. Each of these groups is described below.

The present invention provides a process for the preparation of an imine having the partial structure (I):

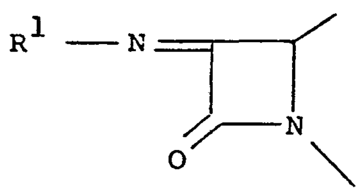

(I)

wherein $R^1$ is a carboxylic acyl group, which process comprises treating a compound having the partial structure (II)

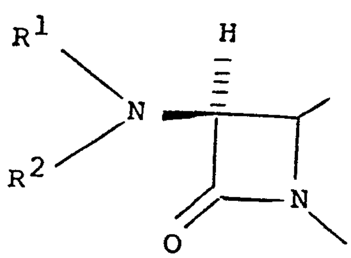

(II)

wherein $R^2$ is a halogen atom, with a non-nucleophilic base.

By 'carboxylic acyl group' herein is meant an aryloxycarbonyl, arylalkyloxycarbonyl, or alkoxycarbonyl group of the formula:

R 0 CO ——

wherein R is aryl, aryl($C_{1-6}$) alkyl or optionally substituted ($C_{1-6}$) alkyl It will therefore be appreciated that the compounds of formula (II) are N-halocarbamates (N-halourethanes).

Suitably the group $R^1$ is a removable amino protecting group of the urethane type which may if desired be removed from a compound of formula (I) or, more suitably, from a product for which the compound of formula (I) serves as an intermediate. Examples of suitable protecting groups of this nature, and conditions for their removal without disruption of the remainder of the molecule are well known in the art.

Suitable removable amino-protecting carboxylic acyl groups for $R^1$ include benzyloxycarbonyl optionally substituted in the phenyl ring by one or two substituents selected from $C_{1-4}$ alkyl, $C_{1-4}$ alkoxy, trifluoromethyl, halogen or nitro; $C_{1-4}$ alkyloxycarbonyl, for example tert-butoxycarbonyl; or $C_{1-4}$ alkyloxycarbonyl optionally substituted in the alkyl group by up to three substituents chosen from $C_{1-4}$ alkoxy, halo or nitro, for example 2,2,2,-trichloroethoxycarbonyl or 1-chloroethoxycarbonyl.

Preferred examples of removable amino-protecting acyl groups within $R^1$ include those listed above which are removable under acid conditions optionally in the presence of a group IIb metal.

Particular carboxylic acyl groups for $R^1$ include 2,2,2- trichloroethoxycarbonyl, ethoxycarbonyl and benzyloxycarbonyl, and 4-nitrobenzyloxycarbonyl.

The term 'halogen' refers to fluorine, chlorine, bromine and iodine.

A preferred halogen atom for $R^2$ is chlorine.

Suitable non-nucleophilic bases for use in the process include 1,8-diazabicyclo [5.4.0]undec-7-ene, 1,5-diazabicyclo [4.3.0] non-5-ene, 1,4-diazabicyclo [2.2.2]octane, dimethylaminopyridine or a tri(lower alkyl) amine.

A preferred base is 1,8-diazabicyclo [5.4.0]undec-7-ene (DBU).

Suitable solvents in which the reaction may be performed include for example, tetrahydrofuran, dimethylformamide, dichloromethane and methylisobutylketone. The reactions are generally carried out under an inert atmosphere and at moderate to low temperature ie in the range -100°C to +30°C.

A suitable example of a compound of partial structure (I) is a compound of formula (III):

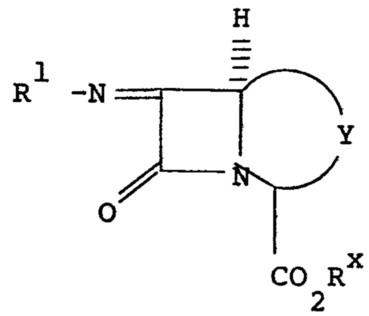

(III)

wherein $R^1$ is as defined hereinbefore; $R^x$ is hydrogen or a readily removable carboxyl protecting group; and Y is:

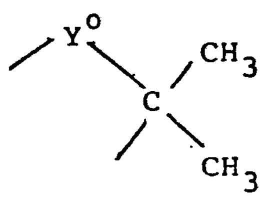 , 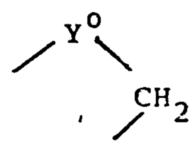 , 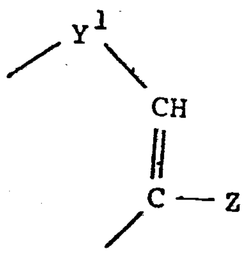 or 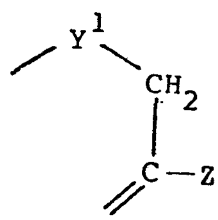

wherein $Y^0$ is sulphur, SO or $SO_2$, $Y^1$ is oxygen, sulphur, SO, $SO_2$ or $-CH_2-$ and Z represents hydrogen, halogen, or an organic group such as $C_{1-4}$ alkoxy, $-CH_2Q$ or -CH=CH-Q wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded <u>via</u> carbon, a heterocyclylthio group or a nitrogen containing heterocyclic group bonded <u>via</u> a nitrogen atom.

Suitable values for Q in the compounds of the formula (III) include the acetoxy, heterocyclylthio group, and nitrogen containing heterocyclic group bonded <u>via</u> nitrogen.

More suitably Q represents the acetoxy or heterocyclylthio group.

The heterocyclylthio group may suitably be represented by the formula:

- S - Het

wherein 'Het' is a five or six membered heterocyclic ring containing from 1 to 4 atoms selected from N, O, and S unsubstituted or substituted with one or two groups selected from $C_{1-6}$ alkyl, $C_{1-6}$ alkoxy, hydroxyalkyl, $C_{1-6}$ alkenyl, alkoxyalkyl, carboxyalkyl, sulphonylalkyl, carbamoylalkyl, trifluoromethyl, hydroxy, halogen, oxo, optionally substituted aminoalkyl, and carboxyalkyl or two substituents may be linked to form the residue of a heterocyclic or carbocyclic ring.

Examples of the group 'Het' include unsubstituted and substituted imidazolyl, triazolyl, tetrazolyl, thiazolyl, thiadiazolyl, thiatriazolyl, oxazolyl, triazinyl and oxadiazolyl.

Suitable groups 'Het' include unsubstituted and substituted 1, 2, 3-triazolyl; 1, 2, 4-triazolyl; tetrazolyl; oxazolyl; thiazolyl; 1, 3, 4-oxadiazolyl; 1, 3, 4-thiadiazolyl; or 1, 2, 4-thiadiazolyl.

Preferably the heterocyclylthio group is
1-methyl-1H-tetrazol-5-ylthio,
2-methyl-1,3,4-thiadiazol-5-ylthio,
1-carboxymethyl-1H-tetrazol-5-ylthio or
6-hydroxy-2-methyl-5-oxo-2H-1,2,4-triazin-3-ylthio.

The nitrogen containing heterocyclic group bonded via nitrogen is suitably a pyridinium group unsubstituted or substituted with one or two groups selected from $C_{1-6}$ alkyl, $C_{1-6}$ alkoxy, hydroxyalkyl, $C_{1-6}$ alkenyl, alkoxyalkyl, carboxyalkyl, sulphonylalkyl, carbamoylmethyl, carbamoyl, trifluoromethyl, hydroxy, halogen, oxo, aminoalkyl, or two substituents on adjacent carbon atoms may form the residue of a carbocyclic ring.

When used herein the term 'carboxylic ester' unless otherwise defined suitably includes $C_{1-6}$ alkyl esters.

When used herein the term 'acyloxy' unless otherwise defined suitably includes $C_{1-6}$ alkylcarbonyloxy groups.

When used herein the term 'aryl' unless otherwise defined suitably includes phenyl and naphthyl, preferably phenyl, optionally substituted with up to five halogen, $C_{1-6}$ alkyl, $C_{1-6}$ alkoxy, halo $(C_{1-6})$ alkyl, hydroxy, amino, carboxy, $C_{1-6}$ alkoxycarbonyl, or $C_{1-6}$ alkoxycarbonyl-$(C_{1-6})$alkyl, $C_{1-6}$ alkylcarbonyloxy or $C_{1-6}$ alkoxycarbonyloxy groups.

When used herein the term 'heterocyclyl' unless otherwise defined suitably includes single or fused aromatic or non-aromatic heterocyclic rings comprising up to four hetero atoms in the ring selected from oxygen, nitrogen, and sulphur and optionally substituted with up to three halogen, $C_{1-6}$ alkyl, $C_{1-6}$ alkoxy, halo-$(C_{1-6})$-alkyl, hydroxy, amino, carboxy, $C_{1-6}$ alkoxycarbonyl, $C_{1-6}$ alkoxycarbonyl$(C_{1-6})$ alkyl, aryl, oxo, carboxy$(C_{1-6})$alkyl, carbamoyl-$C(_{1-6})$alkyl, amino$(C_{1-6})$alkyl, substituted amino$(C_{1-6})$ alkyl or sulphonyl$(C_{1-6})$alkyl groups.

Suitably the heterocyclic ring comprises from 4 to 7 ring atoms, preferably 5 or 6 atoms.

When used herein the term 'lower' indicates that the group contains 1 to 6 carbon atoms.

Suitably Y is:

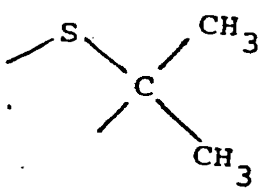
,
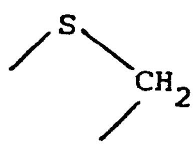
,
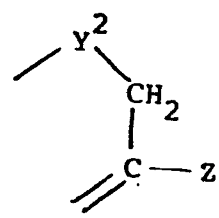

wherein $Y^2$ is oxygen, sulphur or $-CH_2-$ and Z is as hereinbefore defined.

Preferred values for Y in the compounds of formula (III) are $-S-C(CH_3)_2-$ and $-S-CH_2-C(CH_2Q)=$, ie when the compound of formula (III) is a derivative of a penicillin or cephalosporin.

A particularly preferred value for Y is $-S-C(CH_3)_2-$.

A further preferred value for Y is $-S-CH_2-CZ=$ wherein Z is as hereinbefore defined.

The imine of formula (III) is prepared by treatment with base of a compound of formula (IV):

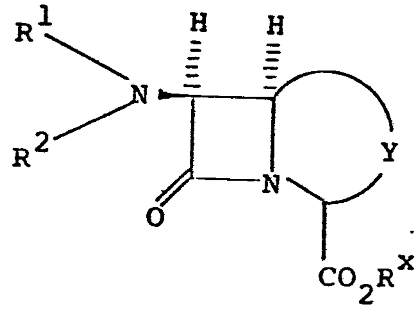

(IV)

wherein $R^1$, $R^2$, $R^x$ and Y are as hereinbefore defined and wherein any reactive groups may be protected.

Suitable compounds within formula (IV) are those of formula (V):

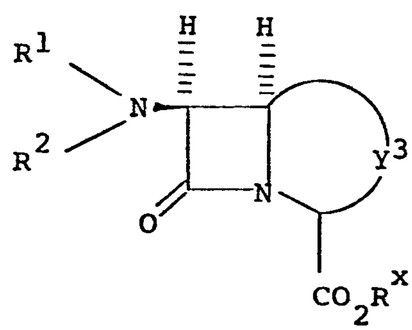

(V)

wherein $R^1$ and $R^x$ are as hereinbefore defined and wherein any reactive groups may be protected; and $Y^3$ is:

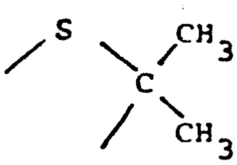

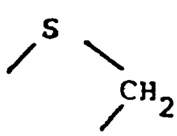

,

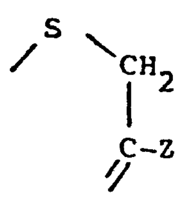

or

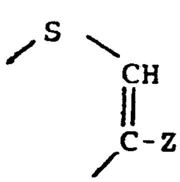

It will also be appreciated that the imine (I) may be generated in a similar manner to that described above from a compound of partial structure (IIA):

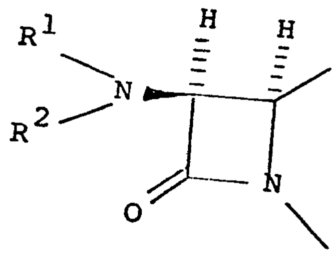

(IIA)

wherein $R^1$ and $R^2$ are as hereinbefore defined.

The imines of formulae (I) and (III) are often unstable intermediates and are conveniently reacted in situ without isolation, to form other β-lactam compounds.

In particular, an imine of partial structure (I) as hereinbefore defined can be converted to a β-lactam having the partial structure (VI):

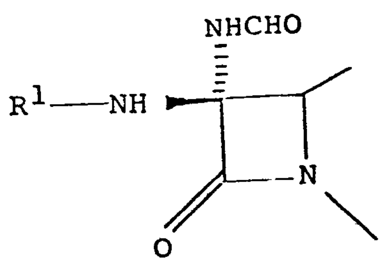

(VI)

wherein $R^1$ is as hereinbefore defined, by reaction with a nucleophilic derivative of formamide, as described in our copending European Patent Application No.84300338.5

Suitable nucleophilic derivatives of formamide include N-silyl, N-stannyl and N-phosphorylated derivatives.

By the term '*N*-silyl derivative' of formamide is meant the product of reaction of the amino group of formamide with a silylating agent such as a halosilane or a silazane of the formula:

$L_3$-Si-U; $L_2$-Si-$U_2$; $L_3$-Si-$NL_2$;

$L_3$-Si-NH-Si-$L_3$; $L_3$-Si-NH-COL; $L_3$-Si- NH-CO-NH-Si-$L_3$;

L-NH-CO-NH-Si-$L_3$; LC-OSi-$L_3$

$NSiL_3$

wherein U is a halogen and the various groups L which may be the same or different, each represent hydrogen, alkyl, alkoxy, aryl, or aralkyl. Preferred silylating agents are silyl chlorides, particularly trimethylchlorosilane.

The term 'N-stannyl derivative' of formamide includes the product of reaction of the amino group of formamide with a stannylating agent such as a halostannane of formula:

$L_3SnU$

wherein L and U are as defined hereinbefore.

The term 'N-phosphorylated' derivative of formamide is intended to include compounds wherein the amino group of formamide is substituted with a group of formula:

$- P.R_j\ R_k$

wherein $R_j$ is an alkyl, haloalkyl, aryl, aralkyl, alkoxy, haloalkoxy, aryloxy, aralkyloxy or dialkylamino group, $R_k$ is the same as $R_j$ or is halogen or $R_j$ and $R_k$ together form a ring.

Other suitable nucleophilic derivatives of formamide include

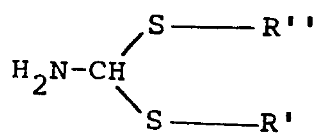

wherein R' and R '' may be the same or different and each represent a $C_{1-6}$ alkyl group or R' and R'' together represent a $C_{2-4}$ alkylene di-radical.

Preferably the nucleophilic derivative of formamide is a N,N-bis (tri-loweralkylsilyl) formamide, in particular N,N-bis(trimethylsilyl)formamide.

Suitable solvents in which the reaction may be performed include for example, tetrahydrofuran, dimethylformamide, dichloromethane and methylisobutylketone. The reactions are generally carried out under an inert atmosphere and at moderate to low temperatures i.e. in the range -100°C to +30°C. The course of the reaction may be followed by conventional methods such as thin layer chromatography and terminated when an optimum quantity of product is present in the reaction mixture.

Subsequent to the reaction it may be necessary to regenerate the formamido group from any derivative; suitable methods include those known in the art such as, for example, acid or base hydrolysis or treatment with a metal ion such as mercury, silver, thallium, lead or copper.

Thus in a further aspect of the invention there is provided a process for the preparation of a compound of partial structure (VI)

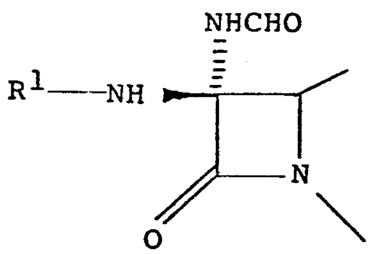

(VI)

wherein $R^1$ is as hereinbefore defined by treating a compound of partial structure (II),

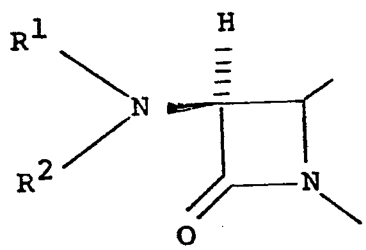

(II)

wherein $R^1$ and $R^2$ are as hereinbefore defined with (i) a non-nucleophilic base and subsequently in situ with (ii) a nucleophilic derivative of formamide.

Suitable non-nucleophilic bases and nucleophilic derivatives of formamide are described hereinbefore.

An example of the above process is the preparation of a compound of formula (VII) or a salt thereof:

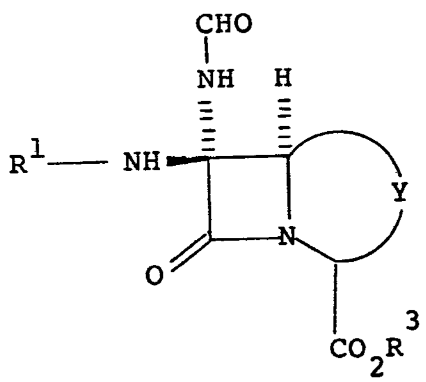

(VII)

wherein $R^1$ and Y are as defined hereinbefore; $R^3$ is hydrogen or a readily removable carboxyl protecting group which process comprises treating an intermediate imine of formula (III) as hereinbefore defined wherein any reactive groups may be protected; with a nucleophilic derivative of formamide and thereafter, if necessary, carrying out one or more of the following steps:

(i) removing any protecting groups;

(ii) converting a group $R^x$ to a group $R^3$;

(iii) converting one group Z into a different group Z;

(iv) converting the product into a salt.

Those compounds of the formula (VII) wherein $R^3$ is a readily removable carboxyl protecting group or a non-pharmaceutically acceptable salt, are useful as intermediates in the preparation of compounds of the formula (VII) wherein $R^3$ is a free carboxyl group or a pharmaceutically acceptable salt thereof. The conversion referred to above may be accomplished by procedures <u>per se</u> well known in the art. Also included within the readily removable carboxyl protecting groups $R^3$ are pharmaceutically acceptable <u>in vivo</u> hydrolysable ester groups.

Examples of suitable pharmaceutically acceptable <u>in vivo</u> hydrolysable ester groups include those which break down readily in the human body to leave the parent acid or its salt. Suitable ester groups of this type include those of part formula (i), (ii) and (iii):

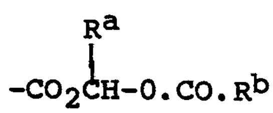
(i)

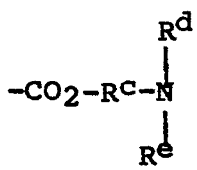
(ii)

$-CO_2CH_2-OR^f$ (iii)

wherein $R^a$ is hydrogen, methyl, or phenyl, $R^b$ is $C_{1-6}$ alkyl, $C_{1-6}$ alkoxy or phenyl; or $R^a$ and $R^b$ together form a 1,2-phenylene group optionally subsituted by one or two methoxy groups; $R^c$ represents $C_{1-6}$ alkylene optionally substituted with a methyl or ethyl group - $R^d$ and $R^e$ independently represent $C_{1-6}$ alkyl; $R^f$ represents $C_{1-6}$ alkyl.

Examples of suitable *in vivo* hydrolysable ester groups include for example acyloxyalkyl groups such as acetoxymethyl, pivaloyloxymethyl, α-acetoxyethyl and α-pivaloyloxyethyl groups; alkoxycarbonyloxyalkyl groups such as ethoxycarbonyloxymethyl and α-ethoxycarbonyloxyethyl; dialkylaminoalkyl especially di-loweralkylamino alkyl groups ;such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

Suitable readily removable carboxyl protecting groups for the group $-CO_2R^3$ in formula (VII) and $-CO_2R^x$ in formula (III) include salt and ester derivatives of the carboxylic acid. The derivative is preferably one which may readily be cleaved.

Suitable ester-forming carboxyl-protecting groups are those which may be removed under conventional conditions. Such groups for $R^3$ and $R^x$ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl,methoxymethyl, a silyl, stannyl or phosphorus-containing group, an oxime radical of formula $-N=CHR^o$ where $R^o$ is aryl or heterocyclyl, or an *in vivo* hydrolysable ester radical such as defined above.

The carboxyl group may be regenerated from any of the above esters by usual methods appropriate to the particular $R^3$ or $R^x$ group, for example, acid - and base - catalysed hydrolysis, or by enzymically-catalysed hydrolysis, or by hydrogenolysis.

Suitable pharmaceutically acceptable salts of the carboxy group of the compounds of formulae (VII) and (III) include metal salts eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylene-diamine, 1-ephenamine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins. Other suitable salts include the lithium and silver salt.

The antibacterial compounds produced by the process of the present invention may be formulated into pharmaceutical compositions according to techniques per se known in the art and administered by conventional routes and dosage rates; for example, as disclosed in US Patent Application No. 401266 and European Patent Application No. 8230382.1 (publication No. 0071395) which are incorporated herein by reference.

Compounds of formula (VI) wherein $R^1$ is as hereinbefore defined serve primarily as valuable intermediates for

the preparation of other β-lactam compounds. Thus, it will be appreciated that removal of the carboxylic acyl amino-protecting group $R^1$ from a compound of partial structure (VI) will give a β-lactam of structure (VIII):

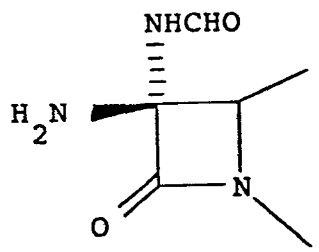

(VIII)

wherein the amino group may be acylated by known techniques to produce β-lactams having the partial structure (IX):

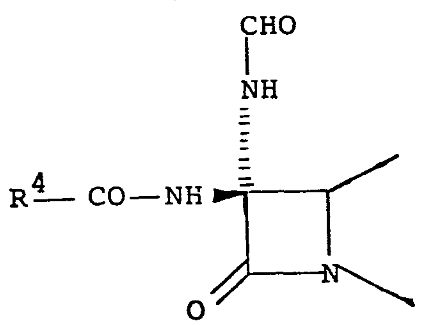

(IX)

wherein $R^4CO$ is an acyl group.

Suitably, removal of a carboxylic acyl amino-protecting group $R^1$ from a compound of structure (VII) or a salt thereof will afford a β-lactam of structure (X):

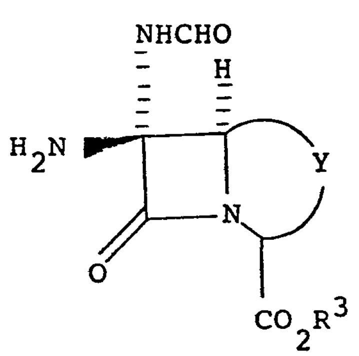

(X)

wherein $R^3$ and Y are as defined hereinbefore.

Methods for preparing compounds of partial structure (IX) wherein $R^4$ is as hereinbefore defined from β-lactams of partial structure (VIII) are described in European Patent Application No. 82303821.1 (Publication Number 0 071 395).

A preferred group of compounds that may be produced from β-lactams of structure (X) by such methods can be represented by the formula (XI) or a pharmaceutically acceptable salt or in vivo hydrolysable ester thereof:

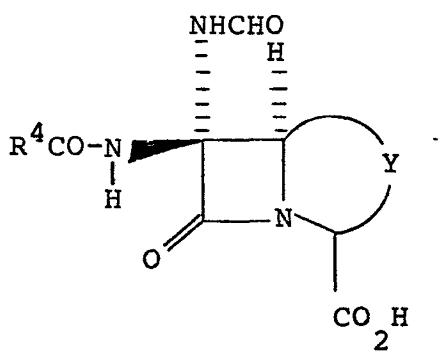

(XI)

wherein Y is as defined with respect to formula (III); and $R^4$ is a group such that $R^4$-CO is an acyl group, in particular an acyl group found in antibacterially active penicillins or cephalosporins.

Compounds of partial structure (II) are obtained by reacting a compound of formula (XII)

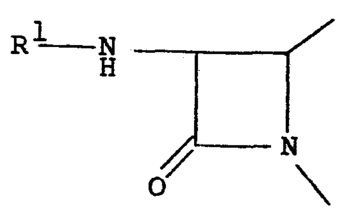

(XII)

wherein $R^1$ is as hereinbefore defined, with base and a source of positive halogen.

A suitable source of halogen is tert-butyl hypochlorite.

Suitable bases include 1,8-diazabicyclo[5.4.0]-undec-7-ene, 1,5-diazabicyclo[4.3.0]non-5-ene, 1,4-diazabicyclo[2.2.2.]octane, dimethylaminopyridine or a tri(lower alkyl) amine.

Suitable solvents in which the reaction may be carried out include for example, tetrahydrofuran, dimethylformamide, dichloromethane and methylisobutylketone. The reactions are generally carried out under an inert atmosphere and at moderate to low temperatures i.e. in the range -100°C to +30°C.

Compounds of formula (IIA), (IV) and (V) can be produced in an analogous manner.

Compounds of formula (XII) are known in the art and may be prepared by conventional methods from compounds of partial structure (XIII).

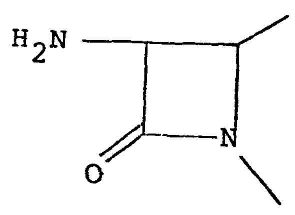

(XIII)

The following Examples illustrate the process of the invention.

Preparation 1

Benzyl 6β-(2,2,2,-trichloroethoxycarbonylamino) penicillanate

Benzyl 6β-aminopenicillanate toluene-4-sulphonate (120g; 0.25 mol) was partitioned between ethyl acetate ($750cm^3$) and saturated aqueous sodium bicarbonate ($1000cm^3$). The phases were separated and the aqueous phase re-extracted with ethyl acetate (2 x $250cm^3$). The combined extracts were washed with water ($500cm^3$) followed by aqueous sodium chloride ($500cm^3$). The organic solution was dried over anhydrous magnesium sulphate, filtered and the filtrates cooled to $-25^oC$. Pyridine ($22cm^3$; 0.28 mol) was added followed by 2,2,2-trichloroethyl chloroformate (54g; 0.256 mol) dropwise over 0.25 hour. The mixture was warmed to ambient temperature during 2 hours and washed sequentially with 1.0M hydrochloric acid (3 x $500cm^3$), water ($500cm^3$), saturated aqueous sodium bicarbonate (3 x $500cm^3$), saturated aqueous sodium chloride and dried (anhydrous magnesium sulphate). The organic solution was filtered and the filtrates concentrated in vacuo to give an oil. Trituration with hexane ($500cm^3$) gave an off-white solid which was recrystallised from 50% ethyl acetate/hexane to give the title compound (90.9g; 74.6%).

The product ran as one component on t.l.c. ($SiO_2$; $60F_{254}$; 50% ethyl acetate/hexane) at an Rf of 0.61 (U.V., 254nm).

νmax (Nujol Mull) 3300, 1770, 1740 and 1545 $cm^{-1}$.
δ($CDCl_3$) 1.42 and 1.62 (6H, 2s, 2-$CH_3$'s), 4.45(1H, s, 3-H), 4.72 (1H, s, $CCl_3$ $\underline{CH_2}$), 5.17 (2H, s, Ph$\underline{CH_2}$O), 5.58 (3H, br m, 5-H, 6-H, CO$\underline{NH}$), 7.33 (5H, s,$C_6H_5$).

Example 1

Benzyl 6α-formamido-6β-(2,2,2-trichloroethoxycarbonylamino)penicillanate

Benzyl 6β-(2,2,2,-trichloroethoxycarbonylamino) penicillanate (1050mg; 2.2mmol) was dissolved in dry tetrahydrofuran at -76° under an atmosphere of nitrogen. 1,8-Diazabicyclo[5.4.0]undec-7-ene (D.B.U.) (304mg; 2mmol) was added and the mixture was stirred for 5 minutes at -76°. Next *tert* butyl hypochlorite (217mg; 2mmol) was added, and the mixture was stirred for a further 5 minutes at -76° before addition of bis(trimethylsilyl) formamide ($0.42cm^3$; 2mmol); the reaction mixture was then warmed to ambient temperature during 2 hours. Ethyl acetate ($20cm^3$) was added and the mixture was washed sequentially with 1M hydrochloric acid (2 x $30cm^3$), water ($30cm^3$), saturated aqueous sodium bicarbonate (2 x $30cm^3$) and saturated sodium chloride (2 x $30cm^3$). The organic solution was dried (anhydrous magnesium sulphate), filtered, and the filtrate reduced *in vacuo* to an oil. The oil was dissolved in diethyl ether ($10cm^3$) and the product crystallised on stirring at 5°. The material was collected by filtration, washed with diethyl ether at 5° and dried *in vacuo* to give the title compound (230mg; 22%).

The material ran as one main component on t.l.c. ($SiO_2$; $60F_{254}$; 50% ethyl acetate/hexane) at an Rf of 0.35 (U.V. 254nm).

νmax (Nujol Mull) 3340, 3160, 1795, 1745, 1670, 1530 and 1495 $cm^{-1}$, δ($CDCl_3$) 1.37 and 1.53 (6H, 2s, 2-$CH_3$'s), 4.52 (1H, s, 3-H), 4.72 (2H, s, $Cl_3C\underline{CH_2}$), 5.17 (2H, s, $Ph\underline{CH_2}O$), 5.66 (1H, s, 5-H), 6.75 (1H, br s, $D_2O$-exchangeable NH), 7.34 (5H, s, $C_6H_5$), 7.70 (1H, br s, $D_2O$-exchangeable NH CHO) and 8.19 (1H, s, NHCHO).

Preparation 2

Benzyl 6β-ethoxycarbonylaminopenicillanate

The title compound was prepared by a similar procedure to that described in Preparation 1, using ethyl chloroformate (2.5g, 0.023 mole). The product was obtained after removal of the solvent, as a viscous oil (6.7g, 89%).

δ($CDCl_3$) 1.27(3H,t,J=7Hz,$\underline{CH_3}CH_2$), 1.40 and 1.65 (6H,2s,2x$CH_3$), 4.00 (2H,q,J=7Hz,$CH_3\underline{CH_2}$), 4.35 (1H,s,3-H), 4.98(2H,s,Ph$\underline{CH_2}$O), 5.1-5.8 (3H,m,5H,6H and CO$\underline{NH}$), 7.15(5H,s,$\underline{Ph}CH_2$O).

Example 2

Benzyl 6β-ethoxycarbonylamino-6α-formamidopenicillanate

Benzyl 6β-ethoxycarbonylaminopenicillanate (1.13g; 0.003 mole) was dissolved in dry tetrahydrofuran (10$cm^3$) and cooled to -65° under an atmosphere of nitrogen. Added D.B.U. (0.45g; 0.003 mole) and the mixture was stirred for 5 minutes at -65°. Added t-butylhypochlorite (0.34g; 0.0032 mole) and stirred for a further 5 minutes at -65°. Added bis(trimethylsilyl)formamide (4.77g; 0.025 mole) and the reaction mixture warmed to ambient temperature during 2 hours. Ethyl acetate (30$cm^3$) and water (30$cm^3$) were added to the reaction mixture. The phases were separated and the organic phase washed sequentially with 0.1M hydrochloric acid (30 $cm^3$), 0.1M sodium bicarbonate (30$cm^3$), water (30$cm^3$) and saturated aqueous sodium chloride (30$cm^3$). The organic soluton was dried (anhydrous magnesium sulphate) filtered and the filtrates reduced in vacuo to an oil. The product was purified by column chromatography on silica G60 (30g), using ethyl acetate - n-hexane (1:1) as eluent. Removal of the solvent in vacuo gave the title compound as a pale yellow foam (0.54g; 43%).

The material ran as one component on t.l.c. (silica, 60F $_{254}$; ethyl acetate-n-hexane, 1:1) at an Rf of 0.10 (u.v. 254 n.m.). $\nu$max(KBr), 3300, 2990, 1780, 1740, 1690, 1510, 1500, 1260$cm^{-1}$.

δ($CDCl_3$), 1.25(3H,m,$\underline{CH_3}CH_2$), 1.38 and 1.55 (6H,2s,2 x $CH_3$), 4.15(2H,m,$CH_3\underline{CH_2}$) 4.54(1H,s,3-H), 5.20(2H,m,Ph$\underline{CH_2}$), 5.68(1H,s,5-H), 6.30(1H,br.s,$D_2O$ exchange,CO$\underline{NH}$), 7.35(5H,s,$\underline{Ph}CH_2$), 7.67(1H,br.s,$D_2O$ exchange, $\underline{NH}$CHO), 8.21(1H,s,NH$\underline{CH}$O).

Preparation 3



Benzyl 6β-benzyloxycarbonylaminopenicillanate

The title compound was prepared by a similar procedure to that described in Preparation 1, using benzyl chloroformate (4.5g; 0.025 mole). The product was obtained as a viscous oil (8.7g; 100%). δ ($CDCl_3$), 1.35 and 1.50(6H,2s,2 x Ph$CH_2$), 5.35(2H,bs,5-H,6-H), 5.6-5.9(1H,bs,CONH), 7.15(10H,bs,2 x Ph$CH_2$).

Example 3

Benzyl 6β-benzyloxycarbonylamino-6α-formamidopenicillanate

Benzyl 6β-benzyloxycarbonylaminopenicillanate (1.32g; 0.003 mole) was dissolved in dry tetrahydrofuran ($10cm^3$) at -65° under an atmosphere of nitrogen. Added D.B.U. (0.45g; 0.003 mole) and the mixture was stirred for 5 minutes at -65°. Added t-butylhypochlorite (0.34g; 0.003 mole) and stirred for a further 5 minutes at -65°. Added bis(trimethylsilyl) formamide (4.77g; 0.025 mole and the reaction mixture warmed to ambient temperature during 2 hours. Ethyl acetate ($30cm^3$) and water ($30cm^3$) were added to the reaction mixture and the phases separated. The organic phase was washed sequentially with 0.1M hydrochloric acid ($30cm^3$), 0.1M hydrochloric acid ($30cm^3$), 0.1M sodium bicarbonate ($30cm^3$), water ($30cm^3$) and saturated aqueous sodium chloride ($30cm^3$). The organic solution was dried (anhydrous magnesium sulphate), filtered and the filtrates reduced in vacuo to an oil.

The product was purified by column chromatography on silica G60 (30g), using ethyl acetate-n-hexane (1:1) as eluent. Removal of the solvent in vacuo gave the title compound as a pale yellow foam (0.94g, 65%).

The material ran as one component on t.l.c. (Silica $60F_{254}$; ethyl acetate-n-hexane, 1:1) at an Rf of 0.10 (u.v. 254nm).

$\nu$max(KBr), 3350, 2965, 1785, 1740, 1690, 1510, 1450, $1260cm^{-1}$.

δ($CDCl_3$), 1.40 and 1.52(6H,2s,2 x $CH_3$), 4.54(1H,s,3-H), 5.0-5.3(4H,m, 2 x Ph$CH_2$), 7.53(1H,bs,$D_2O$ exchange, NHCHO), 8.19(1H,s,NHCHO).

Preparation 4

4-Nitrobenzyl-6β-2',2',2'-trichloroethoxycarbonylaminopenicillanate

The title compound was prepared using a similar procedure to that described in Preparation 1, using 4-nitrobenzyl-6β-aminopenicillanate toluene-4-sulphonic acid salt (13.2g; 0.025M) and 2,2,2-trichloroethyl chloroformate (3.5cm$^3$; 0.025M). The reaction afforded the title compound (11.04g; 86%) as a crisp white foam; δ($CDCl_3$) 1.50 and 1.70 (6H,2s,geminal dimethyl), 4.50(1H,s,C(3)-H), 4.77(2H,s,$Cl_3CC\underline{H}_2$), 5.30(2H,s,Ar-$C\underline{H}_2$), 5.57(2H,m,C(5)-H and C(6)H), 7.52(2H,d,J10Hz, aromatic), 8.22(2H,d,J9Hz, aromatic).

Example 4

4-Nitrobenzyl-6α-formamido-6β-2',2',2'-trichloroethoxycarbonylaminopenicillanate

4-Nitrobenzyl-6β-2',2',2'-trichloroethoxycarbonylaminopenicillanate (1.0g, 0.002M) was dissolved in dry tetrahydrofuran(5cm$^3$) at -65° under an atmosphere of nitrogen. Added DBU (0.3cm$^3$, 0.002M) and the mixture was stirred for 5 minutes at -65°. Added t-butylhypochlorite (0.22cm$^3$, 0.002M) and stirred for a further 5 minutes at -65°. Added bis(trimethylsilyl)formamide (3cm$^3$, 0.014M) and the mixture warmed to ambient temperature during 2 hours. Added ethyl acetate (30cm$^3$) and water (30cm$^3$), separated, and the organic layer washed sequentially with 0.1M hydrochloric acid (30cm$^3$), 0.1M sodium carbonate (30cm$^3$) and saturated sodium chloride (30cm$^3$). The organic solution was dried (anhydrous magnesium sulphate), filtered, and the filtrates evaporated in vacuo to an oil. The oil was chromatographed on silica gel, eluting with ethyl acetate-hexane (1:1). Evaporation of the appropriate fractions afforded the title compound (200mg, 18%) as a pale yellow foam; νmax ($CHCl_3$) 1260, 1315, 1400, 1500, 1640, 1680, 1760, 1840, 1900, 1940, 3300cm$^{-1}$; δ($CDCl_3$), 1.35 and 1.50(6H,2s,geminal dimethyl), 4.52(1H,s,C(3)H), 4.68(2H,s,$Cl_3C\underline{H}_2$), 5.19 and 5.27, B(1H,J13.4Hz) and A(1H,J13,1Hz)(ABq,Ar-$C\underline{H}_2$-),5.68(1H,s,C(5)H), 7.03(1H,s,-N$\underline{H}$CHO), 7.51(2H,d,J8.7Hz, aromatic) 8.15(4H,m,OCON$\underline{H}$, NHC$\underline{H}$O, aromatic).

Preparation 5.

4-Nitrobenzyl 6β-(4-nitrobenzyloxycarbonylamino)penicillanate

The title compound was prepared from 4-nitrobenzyl 6-aminopenicillanate, 4-toluene sulphonate (5.23g; 0.01mole) and 4-nitrobenzyl chloroformate (2.16g; 0.01mole), by a procedure similar to that described in Preparation 1. The product was obtained as an off-white foam (5.2g; 98%).

δ($CDCl_3$), 1.46 and 1.66(6H,2s,2 x $CH_3$), 4.48(1H,s,3-H), 5.19 and 5.27(4H,2s,2 x $NO_2PhCH_2$), 5.36-5.93 and 5.50(3H,s+bm,6-H,5-H, CONH), 7.26-7.65 and 7.96-8.29(8H,2bm,2 x $NO_2PhCH_2$).

The product ran as one main component on t.l.c. at an Rf of 0.31 with small impurities at 0.44 and 0.56. ($SiO_2$; $60F_{254}$; 50% ethyl acetate/n-hexane; u.v. 254nm).

The material was used without further purification.

Example 5.

4-Nitrobenzyl 6α-formamido-6β-(4-nitrobenzyloxycarbonylamino)penicillanate

4-Nitrobenzyl 6β-(4-nitrobenzyloxycarbonylamino)penicillanate (2.65g; 0.005mole) was dissolved in dry tetrahydrofuran ($50cm^3$) and cooled to -65° under an atmosphere of nitrogen. Added DBU ($0.75cm^3$) and the mixture was stirred at -65° for 5 minutes. Added t-butylhypochlorite ($0.585cm^3$) and stirring was continued for a further 5 minutes at -65°. Excess of bis(trimethylsilyl) formamide ($8.0cm^3$) was added and the reaction mixture warmed to ambient temperature during 2.0 hours. The mixture was partitioned between ethyl acetate ($50cm^3$) and water ($50cm^3$). The organic phase was washed sequentially with 1M hydrochloric acid ($50cm^3$), 0.5M sodium bicarbonate ($50cm^3$) and saturated aqueous sodium chloride ($50cm^3$). The solution was dried (anhydrous magnesium sulphate) and concentrated *in vacuo* to a yellow foam.

The title compound was obtained after chromatography on silica G60 (30g), eluting with ethyl acetate/n-hexane (1:1) followed by ethyl acetate, as a pale yellow solid (1.06g; 37%) melting at 92-96°.

ν max(KBr), 3320, 2980, 1783, 1745, 1695, 1610, 1525, $1350cm^{-1}$.

δ($CDCl_3$); 1.42 and 1.56(6H,2s,2 x $CH_3$), 4.58(1H,s,3-H), 5.26(4H,m,2 x $NO_2PhCH_2$) 5.67(1H,s,5-H), 6.48-6.62(1H,bd,$D_2O$ exchange, CONH), 7.53(5H,m,AROM. + NHCHO), 8.23(5H,m,AROM. + NHCHO).

Preparation 6 

Methyl 6β-benzyloxycarbonylaminopenicillanate

6-Aminopenicillanic acid (21.6g; 0.1 mole) was slurried in water ($150cm^3$) and the pH adjusted to 8.5 by addition of 2M sodium hydroxide. Solution cooled to 5° in an ice salt bath. Added benzyl chloroformate (18.7g; 0.11 mole) dropwise, maintaining the pH at 8.5 by concurrent addition of 1M sodium bicarbonate. When the pH was stable the solution was warmed to ambient temperature and stirred for 1 hour. The solution was washed with diethyl ether (2 x $200cm^3$), covered with ethyl acetate ($250cm^3$) and acidified to pH 2.0 with 2M hydrochloric acid. Phases separated. The aqueous phase was further extracted with ethyl acetate ($200cm^3$) and the combined organic phases washed with saturated aqueous sodium chloride (2 x $150cm^3$), dried (anhdyrous magnesium sulphate) and the solvent removed in vacuo to give 6β-benzyloxycarbonylaminopenicillanic acid (35.9g; >100%) as a crisp, white foam.

δ($CDCl_3$), 1.56 and 1.61(6H,2s,2 x $CH_3$), 4.38(1H,s,3-H), 5.08(2H,s,Ph($H_2$), 5.29-5.97(3H,bm,5-H,6-H,CONH), 7.24(5H,s,$PhCH_2$), 10.11(1H,bs,$D_2O$ exchange,$CO_2H$).

The major impurity appeared to be ethyl acetate. The material was used for the next reaction without further purification.

6β -Benzyloxycarbonylaminopenicillanic acid (3.5g; 0.01 mole) was dissolved in dimethylformamide ($20cm^3$), the solution stirred at ambient temperature, and treated with triethylamine (1.52g; 0.015 mole) followed by iodomethane (2.28g; 0.015 mole). The reaction was stirred 1.5 hours at ambient temperature, then diluted with ethyl acetate ($150cm^3$), and water ($150cm^3$). The organic extract was washed with water (2 x $50cm^3$), 0.5M-sodium bicarbonate ($50cm^3$), water ($50cm^3$), saturated aqueous sodium chloride ($25cm^3$) and dried (anhydrous magnesium sulphate). After filtration, the filtrate was reduced in vacuo to give methyl 6β-benzyloxycarbonylaminopenicillanate (2.0g; 55%) as a crisp, yellow-brown foam.

δ($CDCl_3$) 1.45 and 1.63 (6H,2s,2 x $CH_3$), 3.68(3H,s,$CO_2CH_3$), 4.39(1H,s,3-H), 5.05(2H,s,$PhCH_2$), 5.3-5.9(3H,bm,5-H,6-H,CONH), 7.24(5H,s,$PhCH_2$).

Example 6



Methyl 6β-benzyloxycarbonylamino-6α-formamidopenicillanate

Methyl 6β-benzyloxycarbonylaminopenicillanate (1.09g, 0.003 mole) was dissolved in dry tetrahydrofuran ($10cm^3$) and cooled to -65° under an atmosphere of nitrogen. Added D.B.U. (0.45g; 0.003 mole) and the mixture was stirred for 5 minutes at -65°. Added t-butylhypochlorite (0.34g; 0.0032 moles) and stirred for a further 5 minutes at -65°. Added bis(trimethylsilyl) formamide (4.77g; 0.025 moles) and the reaction mixture warmed to ambient temperature during 2 hours. Ethyl acetate ($30cm^3$) and water ($30cm^3$) were added to the reaction mixture. The phases were separated and the organic phase washed sequentially with 0.1M hydrochloric acid ($30cm^3$), 0.1M sodium bicarbonate ($30cm^3$) water ($30cm^3$), and saturated aqueous sodium chloride ($30cm^3$). The organic soluton was dried (anhydrous magnesium sulphate), filtered, and the filtrates reduced in vacuo to an oil. The product was purified by column chromatography on silica G60 (30g) using ethyl acetate-n-hexane (1:1) as eluent. Removal of the solvent in vacuo gave the title compound as a pale yellow foam (0.28g, 23%).

The material ran as one component on t.l.c. (silica, $60F_{254}$; ethyl acetate-n-hexane 1:1) at an Rf of 0.10 (u.v. 254nm).
$\nu$ max (KBr) 3310, 2970, 1785, 1690-1740(b), 1510, 1500, 1265, 1220.

$\delta$($CDCl_3$); 1.45 and 1.53(6H, 2s, 2 x $CH_3$), 3.76(3H, s, $CO_2\underline{CH_3}$), 4.51(1H, s, 3-H), 5.12(2H, m, $Ph\underline{CH_2}$), 5.67(1H, s, 5-H), 6.42(1H, bd, $CO\underline{NH}$), 7.33(5H, s, $\underline{Ph}CH_2$), 7.72(1H, bs, $D_2O$ exchange, $\underline{NH}CHO$), 8.17(1H, s, $NH\underline{CHO}$).

t-Butyl 7β-(2,2,2-trichloroethoxycarbonylamino) cephalosporanate

The title compound was prepared by a similar procedure to that described in Preparation 1 from t-butyl 7β-aminocephalosporanate (5g, 0.015 moles). The product was obtained, after evaporation of the solvent, as a yellow-brown foam (8g, 100%).

The product ran as one component on t.l.c. (silica $60F_{254}$; ethyl acetate-n-hexane 1:1) at an Rf of 0.70 (u.v. 254n.m.).

($CDCl_3$) 1.53(9H,s,$(CH_3)_3C$), 2.05(3H,s,$CH_2OCOCH_3$), 3.45(2H,bs,$CH_2$ at 2-C) 4.45-5.1(5H,m,$Cl_3CCH_2O$, $CH_2OCOCH_3$, 6-H), 5.4-5.75(1H,m,7-H), 6.55(1H,bd,CONH).

Example 7

t-Butyl 7α-formamido-7β-(2,2,2-trichloroethoxycarbonylamino)cephalosporanate

t-Butyl 7β- (2,2,2-trichloroethoxycarbonylamino)cephalosporanate (10g; 0.02 moles) was dissolved in dry tetrahydrofuran ($100cm^3$) and cooled to -60° under an atmosphere of nitrogen. Added D.B.U. (3.05g; 0.02 moles) and the mixture was stirred for 5 minutes at -60°. Added t-butyl hypochlorite (2.04g; 0.019 moles) and stirred for a further 5 minutes at -60°. added bis(trimethylsilyl) formamide (3.8g; 0.02 moles) and the reaction mixture warmed to ambient temperature during 4 hours. ethyl acetate ($100cm^3$) and water ($100cm^3$) were added to the reaction mixture. The phases were separated and the organic phase washed sequentially with 0.5M hydrochloric acid ($25cm^3$), water ($25cm^3$), 0.25M sodium bicarbonate ($25cm^3$), water ($25cm^3$).

and saturated aqueous sodium chloride ($25cm^3$). The organic solution was dried (anhydrous magnesium sulphate), filtered, and the filtrates reduced in vacuo to an oil. The product was purified by column chromatogrphy on silica G60 (150g) using ethyl acetate-n-hexane 1:1 as eluent. Removal of the solvent in vacuo gave an oil which was further purified by column chromatography G60 (35g) using chloroform-methanol-acetic acid (95:5:1) as eluent. Removal of the solvent in vacuo gave the title compound as a pale brown foam (400mg; 3.7%).

The material ran as one component on t.l.c. (silica; $60F_{254}$; ethyl acetate-n-hexane 1:1) at an Rf of 0.15 (u.v. 254nm). $\nu$max($CH_2Cl_2$) 3380, 1790, 1735 and $1700cm^{-1}$. ($CDCl_3$ 1.50(9H,m,$(CH_3)_3$), 2.09(3H,s,$CH_2OCOCH_3$), 3.2-3.65(2H,m,$CH_2$ at 2-C), 4.55-5.2(5H,m,$Cl_3CH_2O$, $CH_2OCOCH_3$, 6-H), 6.67(1H,s,$D_2O$ exch., CONH), 7.70(1H,bs,$D_2O$ exch. NHCHO), 8.26(1H,s,NHCHO).

Example 8



Benzyl 6β-benzyloxycarbonylamino-6α-formamidopenicillanate

Benzyl 6β-benzyloxycarbonylaminopenicillanate (0.82g, 0.00186M) (from Preparation 3) was dissolved in dry tetrahydrofuran ($8cm^3$) at -65° under an atmosphere of nitrogen. Added 1,5-diazabicyclo[4.3.0]-non-5-ene (0.27g; 0.00218 M) and the mixture was stirred for 5 minutes at -65°. Added t-butylhypochlorite (0.24g, 0.00212M) and stirred for a further 5 minutes at -65°. Added bis(trimethylsilyl) formamide (2.5g, 0.013M) and the reaction mixture warmed to ambient temperature during 2 hours.

Ethyl acetate ($25cm^3$) and water ($25cm^3$) were added to the reaction mixture and the phases separated. The organic phase was washed sequentially with 0.1M hydrochloric acid ($25cm^3$), 0.1M sodium bicarbonate ($25cm^3$), water ($25cm^3$) and saturated aqueous sodium chloride ($25cm^3$). The organic solution was dried (anhydrous magnesium sulphate), filtered and the filtrates reduced in vacuo to an oil. The product was purified by column chromatography on silica G60 (10g), using ethyl acetate-n-hexane (1:1) as eluent. Removal of the solvent in vacuo gave the title compound as a pale yellow oil. (0.085g, 9.5%). The material ran as one component on t.l.c. (silica $60F_{254}$; ethyl acetate-n-hexane, 1:1) at an Rf of 0.10 (u.v. 254nm).

The spectral data were identical in all respects to that obtained in example 3.

CLAIMS

1. A process for the preparation of an imine having the partial structure (I):

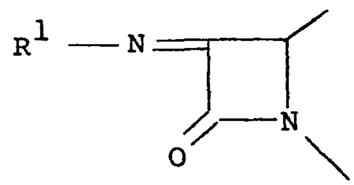

(I)

wherein $R^1$ is a carboxylic acyl group, which process comprises treating a β-lactam having the partial structure (II):

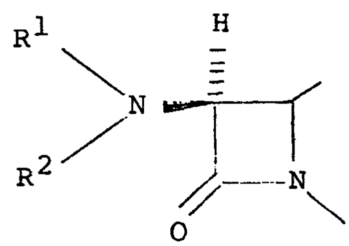

(II)

wherein $R^2$ is a halogen atom with a non-nucleophilic base.

2. A process for the preparation of a compound of formula (III) or a salt thereof:

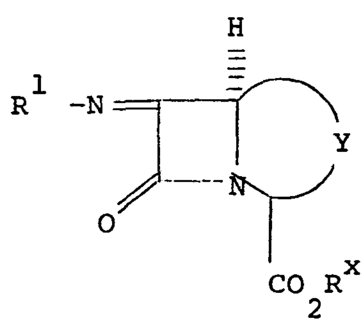

(III)

wherein $R^1$ is a carboxylic acyl group, $R^x$ is hydrogen or a readily removable carboxyl protecting group; and Y is:

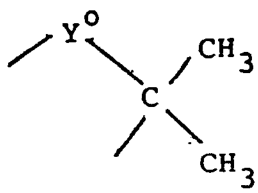

,

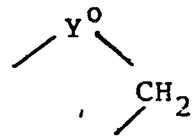

,

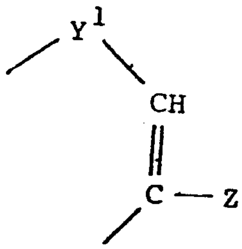

or

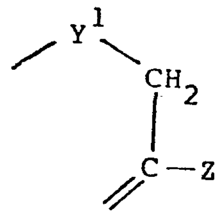

wherein $Y^o$ is sulphur, SO or $SO_2$, $Y^1$ is oxygen, sulphur, SO, $SO_2$ or $-CH_2-$ and Z represents hydrogen, halogen, or an organic group such as $C_{1-4}$ alkoxy, $-CH_2Q$ or $-CH{=}CH-Q$

wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded <u>via</u> carbon, a heterocyclylthio group or a nitrogen-containing heterocyclic group bonded <u>via</u> nitrogen, which process comprises treating an intermediate of formula (IV):

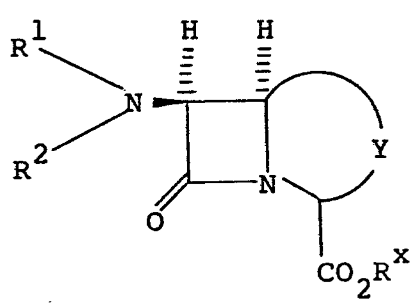

(IV)

wherein $R^2$ is a halogen atom and $R^1$, $R^x$ and Y are as hereinbefore defined; with a non-nucleophilic base.

3. A process as claimed in claim 2 wherein $R^1$ is benzyloxycarbonyl optionally substituted in the phenyl ring by one or two substituents selected from $C_{1-4}$ alkyl, $C_{1-4}$ alkoxy, trifluoromethyl, halogen or nitro, or wherein $R^1$ is $C_{1-4}$ alkyloxycarbonyl optionally substituted in the alkyl group by up to three substituents chosen from $C_{1-4}$ alkoxy, halo or nitro.

4. A process as claimed in Claim 2 or Claim 3 in which $R^1$ is 2,2,2-trichloroethoxycarbonyl, ethoxycarbonyl, benzyloxycarbonyl or 4-nitrobenzyloxycarbonyl.

5. A process as claimed in any one of Claims 2 to 4 wherein $R^2$ is chlorine.

6. A process as claimed in any one of Claims 2 to 5 wherein the non-nucleophilic base is 1,8-diazabicyclo [5.4.0]undec-7-ene.

7. A process as claimed in any one of Claims 2 to 6 wherein Y is:

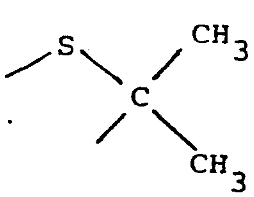 , 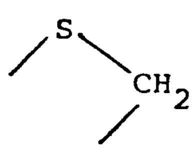 , 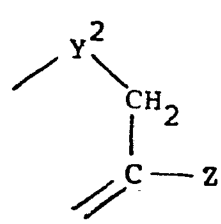

8. A process for the preparation of a compound having the partial structure (VI):

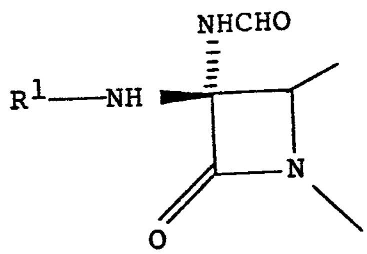

(VI)

wherein $R^1$ is a carboxylic acyl group, which process comprises preparing an imine having the partial structure (I) by a process as claimed in Claim 1 and adding in situ a nucleophilic derivative of formamide.

9. A process for the preparation of a compound of formula (VII) or a salt thereof:

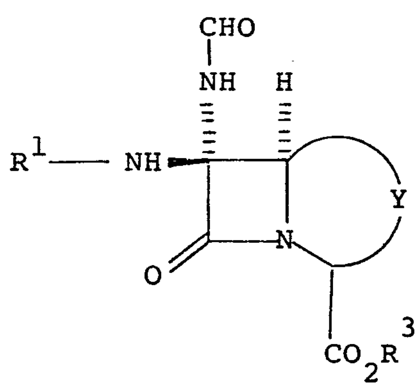

(VII)

wherein any reactive groups may be protected; $R^1$ is a carboxylic acyl group, $R^3$ is hydrogen or a readily removable carboxyl-protecting group; and Y is as defined with respect to formula (III), which process comprises preparing an imine of formula (III) by a process as claimed in any one of Claims 2 to 7 and adding in situ a nucleophilic derivative of formamide; and thereafter, if necessary, carrying out one or more of the following steps:

(i) removing any protecting groups;

(ii) converting a group $R^x$ to a group $R^3$;

(iii) converting one group Z into a different group Z;

(iv) converting the product into a salt.

10. A process as claimed in Claim 9 wherein the nucleophilic derivative of formamide is N,N-bis(tri-loweralkylsilyl)formamide.

11. A process as claimed in Claim 9 or Claim 10 wherein the nucleophilic derivative of formamide is N,N-bis-trimethylsilylformamide.

12. A process for the preparation of a compound of formula (X) or a salt thereof:

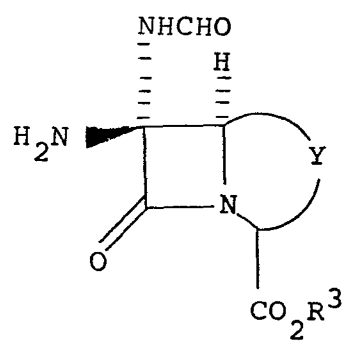

(X)

wherein

$R^3$ is hydrogen or a readily removable carboxy protecting group; and Y is as defined with respect to formula (III), which process comprises preparing a compound of formula (VII) by the process as claimed in any one of Claims 9 to 11, thereafter removing to amino-protecting group $R^1$ and finally, if necessary, carrying out one or more of the following steps:

(i) removing any further protecting groups;

(ii) converting a group $R^x$ into a group $R^3$;

(iii) converting one group Z into a different group Z;

(iv) converting the product into a salt.